## Europäisches Patentamt
## European Patent Office
## Office européen des brevets

(19)

(11) Veröffentlichungsnummer: **0 125 575**

A2

(12)

# EUROPÄISCHE PATENTANMELDUNG

(21) Anmeldenummer: 84105023.0

(22) Anmeldetag: 04.05.84

(51) Int. Cl.³: **C 07 C 67/58**
C 07 C 69/14, C 07 C 29/86
C 07 C 31/08, C 07 C 31/10
C 07 C 31/12

(30) Priorität: 09.05.83 DE 3316930

(43) Veröffentlichungstag der Anmeldung:
21.11.84 Patentblatt 84/47

(84) Benannte Vertragsstaaten:
BE DE FR GB IT NL

(71) Anmelder: HOECHST AKTIENGESELLSCHAFT
Postfach 80 03 20
D-6230 Frankfurt am Main 80(DE)

(72) Erfinder: Leupold, Ernst Ingo, Dr.
Am Zäunefeld 15
D-6392 Neu-Anspach(DE)

(72) Erfinder: Wojtech, Bernhard, Dr.
Kelkheimer Strasse 67
D-6232 Bad Soden am Taunus(DE)

(72) Erfinder: Schmidt, Hans-Joachim, Dr.
Am Burgenblick 6
D-6240 Königstein/Taunus(DE)

(54) Verfahren zur extraktiven Trennung eines alkohol- und esterhaltigen Gemischs.

(57) Die Erfindung betrifft ein Verfahren zur extraktiven Trennung eines alkohol- und esterhaltigen Gemischs aus mindestens drei Komponenten. Die Komponenten sind dabei beliebige Mitglieder der aus den $C_2$- bis $C_4$-Alkoholen und ihren Essigsäureestern bestehenden Klasse. Man erhält eine Alkoholfraktion und eine Esterfraktion.

EP 0 125 575 A2

Croydon Printing Company Ltd.

Verfahren zur extraktiven Trennung eines alkohol- und esterhaltigen Gemischs

Die vorliegende Erfindung betrifft ein Verfahren zur extraktiven Trennung eines alkohol- und esterhaltigen Gemischs aus mindestens drei Komponenten der aus den $C_2$- bis $C_4$-Alkoholen und ihren Essigsäureestern bestehenden Klasse in eine Alkohol- und eine Esterfraktion.

Bei der CO-Hydrierung an heterogenen Katalysatoren gemäß den deutschen Offenlegungsschriften 2.628.463 und 2.748.097 entstehen Gemische sauerstoffhaltiger Verbindungen, die sich durch Destillation in verschiedene Fraktionen auftrennen lassen. Eine dieser Fraktionen enthält neben Wasser Alkohole mit 2 bis 4 Kohlenstoffatomen sowie deren Essigsäureester. Die weitergehende destillative Auftrennung dieser Fraktion wird durch die Azeotropbildung der einzelnen Komponenten verhindert.

Die extraktive Abtrennung beispielsweise des Ethanols aus seinem Gemisch mit Essigsäureethylester ist zwar bekannt, jedoch gab es bisher kein Verfahren, nach dem ein alkohol- und esterhaltiges Gemisch aus mindestens drei Komponenten praktisch quantitativ in eine Alkohol- und eine Esterfraktion getrennt werden kann.

Es wurde nun ein Verfahren zur extraktiven Trennung eines alkohol- und esterhaltigen Gemischs aus mindestens drei Komponenten der aus den $C_2$- bis $C_4$-Alkoholen und ihren Essigsäureestern bestehenden Klasse in eine Alkohol- und eine Esterfraktion gefunden, das dadurch gekennzeichnet ist, daß man das zu trennende Gemisch in eine mittlere Stufe eines Extraktors, der sich damit in eine Extraktionszone und eine Waschzone aufteilt, einleitet, während man der Endstufe der Waschzone Wasser und im Gegenstrom der Endstufe der Extraktionszone einen aliphatischen Kohlenwasserstoff mit einem Siedepunkt oberhalb von 120°C zuführt, wobei die Alkoholfraktion in Wasser gelöst die Extraktionszone verläßt und die Esterfraktion in dem Kohlenwasserstoff gelöst die Waschzone verläßt.

Nach dem erfindungsgemäßen Verfahren lassen sich alle Gemische, die neben Alkoholen mit 2 bis 4 Kohlenstoffatomen noch Essigsäureester solcher Alkohole enthalten, praktisch vollständig durch Extraktion in die Alkohole einerseits und die Ester andererseits trennen. Man erhält eine wäßrige Phase, aus der sich die Alkohole durch bekannte destillative Verfahren abtrennen lassen, sowie eine organische Phase, aus der sich die Ester destillativ gewinnen lassen, wobei der Kohlenwasserstoff im Sumpf der Destillation anfällt und zur Extraktion recycliert werden kann. Vorzugsweise verwendet man n-Dekan.

Die zur Extraktion eingesetzte Speiselösung enthält einerseits Alkohole mit 2 bis 4 Kohlenstoffatomen, also Ethanol und/oder die isomeren Propanole und/oder Butanole, und andererseits Essigsäureester solcher Alkohole. Dabei können Gemische mit allen genannten Komponenten zum Einsatz kommen. Die Speiselösung kann aber auch weniger Komponenten enthalten. Der oder die anwesenden Ester müssen nicht notwendigerweise die Ester der anwesenden Alkohole sein. Jedoch sind immer mindestens drei Komponenten anwesend. Mischungen, die nur aus Alkoholen oder nur aus Estern bestehen, sind ausgeschlossen. Eine Dreikomponentenmischung enthält daher entweder zwei Alkohole und einen Ester, oder einen Alkohol und zwei Ester. Besonders wichtig ist die Mischung aus Ethanol, n-Propanol, n-Butanol und den drei entsprechenden Acetaten.

Die Mengenverhältnisse der einzelnen Alkohole und Ester können in weiten Grenzen schwanken.
Ein bereits vorhandener Wassergehalt reduziert den für die Extraktion notwendigen Wasserzusatz.

Als Extraktoren können allgemein bekannte Apparate, wie Mischabsetzer oder Extraktionskolonnen, eingesetzt werden. Das Mengenverhältnis Kohlenwasserstoff/Wasser/Speiselösung ist variabel. Geeignet sind Verhältnisse von 50 - 200/30 - 300/100, vorzugsweise 120 - 180/170 - 280/100.

Zur Verbesserung der Trennwirkung können dem Wasser Lipophilierungsmittel, wie Ethylenglykol oder Dimethylformamid, in Mengen bis zu 40 Gew.-%, vorzugsweise 15 - 15 Gew.-%, zugesetzt werden.

Die folgenden Beispiele sollen die Erfindung illustrieren. Die dort angegebenen Prozentgehalte sind Gewichtsprozente.

Beispiel 1

In einen Mischabsetzer mit 13 theoretischen Stufen (siehe Figur) werden in die eine Endstufe (Stufe 1) n-Dekan, in die andere Endstufe (Stufe 13) Wasser und in die Stufe 6 das zu trennende Alkohol-Ester-Gemisch (Speiselösung) kontinuierlich bei Raumtemperatur eindosiert. Die Speiselösung hat die Zusammensetzung: 25 % Ethanol (E), 9 % Propanol (P), 7 % n-Butanol (B), 17 % Ethylacetat (Eac), 13 % Propylacetat (Pac), 4 % Butylacetat (Bac). Die pro Minute eingegebenen Mengen n-Dekan/Wasser/Speiselösung betragen 136/235/100 Gramm. Aus Stufe 1 wird 307 g wäßrige Phase pro Minute entnommen. Sie enthält 8,2 % E, 2,9 % P, 2,3 % B und 0,26 % Eac. Aus Stufe 13 wird 164 g n-Dekanphase pro Minute entnommen; sie enthält 9,9 % Eac, 7,9 % Pac, 2,4 % Bac und keine Alkohole. Die Esterausbeute in der Dekanphase beträgt 97,4 %, die Alkoholausbeute in der wäßrigen Phase 100 %.

Beispiel 2

In die gleiche Extraktionsapparatur wie in Beispiel 1 werden folgende Grammengen pro Minute eindosiert: n-Dekan/Wasser/Speiselösung = 168/234/100. Die aus Stufe 1 austretende wäßrige Phase (298 g pro Minute) enthält 8,4 % E, 3,0 % P, 2,4 % B und 0,1 % Eac. Die aus Stufe 13 austretende alkoholfreie Dekanphase (204 g/min.) enthält 8,1 % Eac, 6,4 % Pac, 2,0 % Bac. Das entspricht einer Esterausbeute in der Dekanphase von 99 % und einer Alkoholausbeute in der wäßrigen Phase von 100 %.

Beispiel 3

Man geht vor wie in Beispiel 1, jedoch betragen jetzt die pro Minute eingegebenen Grammengen Dekan/Wasser/Speiselösung = 137/186/100. Die austretenden Phasen enthalten: Wäßrige Phase (252 g/min.) 9,9 % E, 3,6 % P, 2,1 % B und kein Ester; Dekanphase (171 g/min.) 9,9 % Eac, 7,6 % Pac, 2,3 % Bac und 1 % B. Das ergibt eine Esterausbeute von 100 % und eine Alkoholausbeute von 95,9 %.

Beispiel 4

In die gleiche Extraktionsanordnung wie im Beispiel 1 wird statt Wasser eine wäßrige Lösung, die 20 Gew.-% Ethylenglykol als Lipophilierungsmittel enthält, in die Stufe 13 eingespeist. Pro Minute werden folgende Grammengen eingegeben: n-Dekan/wäßrige Lösung/Speiselösung = 137/191/100. Die austretenden Phasen enthalten: wäßrige Phase (259 g/min.) 9,7 % E, 3,4 % P, 2,5 % B, 0,1 % Eac; Dekanphase (169 g/min.) 9,8 % Eac, 7,7 % Pac, 2,4 % Bac, 0,1 % P, 0,3 % B. Das ergibt eine Esterausbeute in der Dekanphase von 98,9 % und eine Alkoholausbeute in der wäßrigen Phase von 98,5 %.

Beispiel 5

In die gleiche Extraktionsanordnung wie im Beispiel 1 wird statt Wasser eine wäßrige Lösung, die 20 Gew.-% Formamid als Lipophilierungsmittel enthält, in die Stufe 13 eingespeist. Die pro Minute eingegebenen Grammengen n-Dekan/ wäßrige Lösung/Speiselösung betragen 137/191/100. Die austretenden Phasen enthalten: wäßrige Phase (262 g/min.) 9,6 % E, 3,4 % P, 2,7 % B, 0,6 % Eac; Dekanphase (166 g/min.) 9,2 % Eac, 7,8 % Pac, 2,4 % Bac. Die Esterausbeute in der Dekanphase beträgt demnach 94,7 %, die Alkoholausbeute in der wäßrigen Phase 100 %.

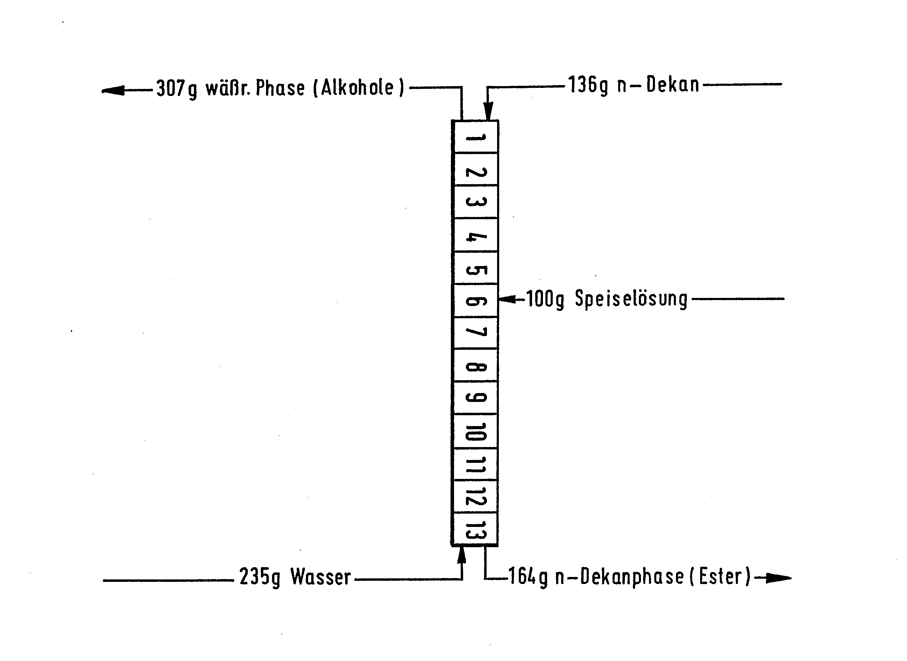

## Patentansprüche:

1. Verfahren zur extraktiven Trennung eines alkohol- und esterhaltigen Gemischs aus mindestens drei Komponenten der aus den $C_2$- bis $C_4$-Alkoholen und ihren Essigsäure-estern bestehenden Klasse in eine Alkohol- und eine Esterfraktion, dadurch gekennzeichnet, daß man das zu trennende Gemisch in eine mittlere Stufe eines Extraktors, der sich damit in eine Extraktionszone und eine Waschzone aufteilt, einleitet, während man der Endstufe der Waschzone Wasser und im Gegenstrom der Endstufe der Extraktionszone einen aliphatischen Kohlenwasserstoff mit einem Siedepunkt oberhalb von 120°C zuführt, wobei die Alkoholfraktion in Wasser gelöst die Extraktionszone verläßt und die Esterfraktion in dem Kohlenwasserstoff gelöst die Waschzone verläßt.

2. Verfahren nach Anspruch 1, dadurch gekennzeichnet, daß als aliphatischer Kohlenwasserstoff n-Dekan eingesetzt wird.

3. Verfahren nach Anspruch 1, dadurch gekennzeichnet, daß zur Erhöhung der Trennleistung dem Wasser Lipophilie-rungsmittel, vorzugsweise Ethylenglykol oder Dimethyl-formamid, zugesetzt werden.